# FASCICULE DE BREVET EUROPEEN

(11) **EP 1 285 067 B1**
(45) Date de publication et mention de la délivrance du brevet: **26.09.2012**
(21) Numéro de dépôt: 01940630.5
(22) Date de dépôt: 29.05.2001
(51) Int. Cl.: C12N 15/29, C07K 14/415, A61K 39/36, G01N 33/50, A01H 5/00

(54) **Clonage et sequençage de l'allergène dac g5 du pollen de Dactylis glomerata, sa préparation et son utilisation**
Klonierung und Sequenzierung des Allergens dac g5 aus dem Pollen von Dactylis glomerata, dessen Herstellung und Verwendung
Cloning and sequencing of dac g5 allergen of Dactylis glomerata pollen, preparation and use thereof

(30) Priorité: 29.05.2000 FR 0006857
(43) Date de publication de la demande: 26.02.2003
(73) Titulaire: Seita Groupe Altadis, 75639 Paris Cedex 13 (FR); STALLERGENES SA, 92183 Antony Cedex (FR)
(72) Inventeur: VAN REE, Ronald, NL-1066 GN Amsterdam (NL); VAN OORT, Erica, NL-3451 TD Vleuten (NL); BONNEAU, Caroline, F-49320 Saint-Saturnin-Sur-Loire (FR); FAYE, Loic, F-76160 Saint-Jacques-sur-Darnetal (FR); GOMORD, Véronique, F-76000 Rouen (FR)
(74) Mandataire: Jacobson, Claude
(86) Numéro de dépôt international: PCT/FR2001/001666
(87) Numéro de publication internationale: WO 2001/092532

(56) Documents cités:
- WO-A-93/04174
- WO-A-95/06728
- MATTHIESEN F ET AL: "GROUP V ALLERGENS IN GRASS POLLENS II. INVESTIGATION OF GROUP V ALLERGENS IN POLLENS FROM 10 GRASSES", CLINICAL AND EXPERIMENTAL ALLERGY, vol. 21, no. 3, 1991, pages 309-320, ISSN: 0954-7894

### LISTE DE SEQUENCES

<110> SEITA
   STALLERGENE
<120> Clonage et séquençage de l'allergène Dac g5 du pollen de Dactylis glomerata, sa préparation et son utilisation
<130> 7770PCTDACG5
<140> XXXXX
   <141> 2001-05-29
<150> FR00/06857
   <151> 2000-05-29
<160> 12
<170> PatentIn Ver. 2.1
<210> 1
   <211> 873
   <212> ADN
   <213> Dactylis glomerata
<220>
   <221> CDS
   <222> (1) .. (873)
   <223> Dac g5 pro-séquence
<400> 1
   atg gcg gtc cag aag tac acc gtg gct cta ttc ctc gcc gtg gtc ctg    48
   Met Ala Val Gln Lys Tyr Thr Val Ala Leu Phe Leu Ala Val Val Leu
     1               5                  10                  15
   gta gcg ggc ccg gtc gcc tcc tac gcc gcc gac gcc ggc tac acc ccg    96
   Val Ala Gly Pro Val Ala Ser Tyr Ala Ala Asp Ala Gly Tyr Thr Pro
                20                  25                  30
   gcc gcc gcg gcc acc ccg gct acc gct gga ggg aag gcg atg acc gag    144
   Ala Ala Ala Ala Thr Pro Ala Thr Ala Gly Gly Lys Ala Met Thr Glu
            35                  40                  45
   gag cag acg cta atc gag gac gtc aat gct ggt ttc aag gcg gcc gtg    192
   Glu Gln Thr Leu Ile Glu Asp Val Asn Ala Gly Phe Lys Ala Ala Val
        50                  55                  60
   gcc gcc gcc tcc agt gcc cct ccg gcg gac aag ttc aag acc ttc gag    240
   Ala Ala Ala Ser Ser Ala Pro Pro Ala Asp Lys Phe Lys Thr Phe Glu
    65                  70                  75                  80
   gcc acc ttc act gcg gcc tgc aag gct aac atc gcc gcc gcc gcc acc    288
   Ala Thr Phe Thr Ala Ala Cys Lys Ala Asn Ile Ala Ala Ala Ala Thr
                    85                  90                  95
   aag gtg ccc ctg ttc gtc gcc aag ctc gac gcc gcc tac gcc gtc gcc    336
   Lys Val Pro Leu Phe Val Ala Lys Leu Asp Ala Ala Tyr Ala Val Ala
               100                 105                 110
   tac aag acc gcc acg ggc ccc acc ccc gag gcc aag tac gac gcc ttc    384
   Tyr Lys Thr Ala Thr Gly Pro Thr Pro Glu Ala Lys Tyr Asp Ala Phe
           115                 120                 125
   gtc gcc gcc ctc acc gaa gcg ctc cgc gtt atc gcc ggc gcc ctc gaa    432
   Val Ala Ala Leu Thr Glu Ala Leu Arg Val Ile Ala Gly Ala Leu Glu
       130                 135                 140
   gtc cat gcc gtc aag ccc gct gcc gag gag gtt ccc gcg gcc aag atc    480
   Val His Ala Val Lys Pro Ala Ala Glu Glu Val Pro Ala Ala Lys Ile
   145                 150                 155                 160
   ccc gcc ggt gag ctg cag att gtc gac aag atc gac gcc gcc tac aag    528
   Pro Ala Gly Glu Leu Gln Ile Val Asp Lys Ile Asp Ala Ala Tyr Lys
                   165                 170                 175
   atc gca gcc acc gct gca aac gcc gcc ccc gcc aac gac aag ttc acc    576
   Ile Ala Ala Thr Ala Ala Asn Ala Ala Pro Ala Asn Asp Lys Phe Thr
               180                 185                 190
   gtc ttc gag ggc gcc ttc aac aag gcc atc aag gag agc acc ggc ggc    624
   Val Phe Glu Gly Ala Phe Asn Lys Ala Ile Lys Glu Ser Thr Gly Gly
           195                 200                 205
   gca tac gag agt tac aag ttc atc ccc acg ctt gag gcc gcg gtc aag    672
   Ala Tyr Glu Ser Tyr Lys Phe Ile Pro Thr Leu Glu Ala Ala Val Lys
       210                 215                 220
   cag gct tac gcc gcc acc gtg gcc gcc gcg ccg gag gtc aag tac gcc    720
   Gln Ala Tyr Ala Ala Thr Val Ala Ala Ala Pro Glu Val Lys Tyr Ala
   225                 230                 235                 240
   gtc ttt gag gcc gcg ctg acc aag gcc atc acc gcc atg tcc gag gca    768
   Val Phe Glu Ala Ala Leu Thr Lys Ala Ile Thr Ala Met Ser Glu Ala
                   245                 250                 255
   cag aag gtc gcc acg cct gcc gcc gtt gct aca ggt gcg gca acc gcc    816
   Gln Lys Val Ala Thr Pro Ala Ala Val Ala Thr Gly Ala Ala Thr Ala
               260                 265                 270
   gct gcc agt gct gct acc ggc gct gcc acc gcc gct gcc ggt ggc tac    864
   Ala Ala Ser Ala Ala Thr Gly Ala Ala Thr Ala Ala Ala Gly Gly Tyr
           275                 280                 285
   aaa gtc tga                                                        873
   Lys Val
       290
<210> 2
   <211> 290
   <212> PRT
   <213> Dactylis glomerata
<400> 2
   Met Ala Val Gln Lys Tyr Thr Val Ala Leu Phe Leu Ala Val Val Leu
    1               5                  10                  15
   Val Ala Gly Pro Val Ala Ser Tyr Ala Ala Asp Ala Gly Tyr Thr Pro
               20                  25                  30
   Ala Ala Ala Ala Thr Pro Ala Thr Ala Gly Gly Lys Ala Met Thr Glu
           35                  40                  45
   Glu Gln Thr Leu Ile Glu Asp Val Asn Ala Gly Phe Lys Ala Ala Val
       50                  55                  60
   Ala Ala Ala Ser Ser Ala Pro Pro Ala Asp Lys Phe Lys Thr Phe Glu
   65                  70                  75                  80
   Ala Thr Phe Thr Ala Ala Cys Lys Ala Asn Ile Ala Ala Ala Ala Thr
                   85                  90                  95
   Lys Val Pro Leu Phe Val Ala Lys Leu Asp Ala Ala Tyr Ala Val Ala
               100                 105                 110
   Tyr Lys Thr Ala Thr Gly Pro Thr Pro Glu Ala Lys Tyr Asp Ala Phe
           115                 120                 125
   Val Ala Ala Leu Thr Glu Ala Leu Arg Val Ile Ala Gly Ala Leu Glu
       130                 135                 140
   Val His Ala Val Lys Pro Ala Ala Glu Glu Val Pro Ala Ala Lys Ile
   145                 150                 155                 160
   Pro Ala Gly Glu Leu Gln Ile Val Asp Lys Ile Asp Ala Ala Tyr Lys
                   165                 170                 175
   Ile Ala Ala Thr Ala Ala Asn Ala Ala Pro Ala Asn Asp Lys Phe Thr
               180                 185                 190
   Val Phe Glu Gly Ala Phe Asn Lys Ala Ile Lys Glu Ser Thr Gly Gly
           195                 200                 205
   Ala Tyr Glu Ser Tyr Lys Phe Ile Pro Thr Leu Glu Ala Ala Val Lys
       210                 215                 220
   Gln Ala Tyr Ala Ala Thr Val Ala Ala Ala Pro Glu Val Lys Tyr Ala
   225                 230                 235                 240
   Val Phe Glu Ala Ala Leu Thr Lys Ala Ile Thr Ala Met Ser Glu Ala
                   245                 250                 255
   Gln Lys Val Ala Thr Pro Ala Ala Val Ala Thr Gly Ala Ala Thr Ala
               260                 265                 270
   Ala Ala Ser Ala Ala Thr Gly Ala Ala Thr Ala Ala Ala Gly Gly Tyr
           275                 280                 285
   Lys Val
       290
<210> 3
   <211> 798
   <212> ADN
   <213> Dactylis glomerata
<220>
   <221> CDS
   <222> (1)..(798)
   <223> Dac g5 séquence mature
<400> 3
   gcc gac gcc ggc tac acc ccg gcc gcc gcg gcc acc ccg gct acc gct    48
   Ala Asp Ala Gly Tyr Thr Pro Ala Ala Ala Ala Thr Pro Ala Thr Ala
    1               5                  10                  15
   gga ggg aag gcg atg acc gag gag cag acg cta atc gag gac gtc aat    96
   Gly Gly Lys Ala Met Thr Glu Glu Gln Thr Leu Ile Glu Asp Val Asn
               20                  25                  30
   gct ggt ttc aag gcg gcc gtg gcc gcc gcc tcc agt gcc cct ccg gcg   144
   Ala Gly Phe Lys Ala Ala Val Ala Ala Ala Ser Ser Ala Pro Pro Ala
           35                  40                  45
   gac aag ttc aag acc ttc gag gcc acc ttc act gcg gcc tgc aag gct   192
   Asp Lys Phe Lys Thr Phe Glu Ala Thr Phe Thr Ala Ala Cys Lys Ala
       50                  55                  60
   aac atc gcc gcc gcc gcc acc aag gtg ccc ctg ttc gtc gcc aag ctc   240
   Asn Ile Ala Ala Ala Ala Thr Lys Val Pro Leu Phe Val Ala Lys Leu
   65                  70                  75                  80
   gac gcc gcc tac gcc gtc gcc tac aag acc gcc acg ggc ccc acc ccc   288
   Asp Ala Ala Tyr Ala Val Ala Tyr Lys Thr Ala Thr Gly Pro Thr Pro
                       85                  90                  95
   gag gcc aag tac gac gcc ttc gtc gcc gcc ctc acc gaa gcg ctc cgc    336
   Glu Ala Lys Tyr Asp Ala Phe Val Ala Ala Leu Thr Glu Ala Leu Arg
               100                 105                 110
   gtt atc gcc ggc gcc ctc gaa gtc cat gcc gtc aag ccc gct gcc gag    384
   Val Ile Ala Gly Ala Leu Glu Val His Ala Val Lys Pro Ala Ala Glu
           115                 120                 125
   gag gtt ccc gcg gcc aag atc ccc gcc ggt gag ctg cag att gtc gac    432
   Glu Val Pro Ala Ala Lys Ile Pro Ala Gly Glu Leu Gln Ile Val Asp
       130                 135                 140
   aag atc gac gcc gcc tac aag atc gca gcc acc gct gca aac gcc gcc    480
   Lys Ile Asp Ala Ala Tyr Lys Ile Ala Ala Thr Ala Ala Asn Ala Ala
   145                 150                 155                 160
   ccc gcc aac gac aag ttc acc gtc ttc gag ggc gcc ttc aac aag gcc    528
   Pro Ala Asn Asp Lys Phe Thr Val Phe Glu Gly Ala Phe Asn Lys Ala
                   165                 170                 175
   atc aag gag agc acc ggc ggc gca tac gag agt tac aag ttc atc ccc    576
   Ile Lys Glu Ser Thr Gly Gly Ala Tyr Glu Ser Tyr Lys Phe Ile Pro
               180                 185                 190
   acg ctt gag gcc gcg gtc aag cag gct tac gcc gcc acc gtg gcc gcc    624
   Thr Leu Glu Ala Ala Val Lys Gln Ala Tyr Ala Ala Thr Val Ala Ala
           195                 200                 205
   gcg ccg gag gtc aag tac gcc gtc ttt gag gcc gcg ctg acc aag gcc    672
   Ala Pro Glu Val Lys Tyr Ala Val Phe Glu Ala Ala Leu Thr Lys Ala
       210                 215                 220
   atc acc gcc atg tcc gag gca cag aag gtc gcc acg cct gcc gcc gtt    720
   Ile Thr Ala Met Ser Glu Ala Gln Lys Val Ala Thr Pro Ala Ala Val
   225                 230                 235                 240
   gct aca ggt gcg gca acc gcc gct gcc agt gct gct acc ggc gct gcc    768
   Ala Thr Gly Ala Ala Thr Ala Ala Ala Ser Ala Ala Thr Gly Ala Ala
                   245                 250                 255
   acc gcc gct gcc ggt ggc tac aaa gtc tga                            798
   Thr Ala Ala Ala Gly Gly Tyr Lys Val
               260                 265
<210> 4
   <211> 265
   <212> PRT
   <213> Dactylis glomerata
<400> 4
   Ala Asp Ala Gly Tyr Thr Pro Ala Ala Ala Ala Thr Pro Ala Thr Ala
    1               5                  10                  15
   Gly Gly Lys Ala Met Thr Glu Glu Gln Thr Leu Ile Glu Asp Val Asn
               20                  25                  30
   Ala Gly Phe Lys Ala Ala Val Ala Ala Ala Ser Ser Ala Pro Pro Ala
           35                  40                  45
   Asp Lys Phe Lys Thr Phe Glu Ala Thr Phe Thr Ala Ala Cys Lys Ala
       50                  55                  60
   Asn Ile Ala Ala Ala Ala Thr Lys Val Pro Leu Phe Val Ala Lys Leu
   65                  70                  75                  80
   Asp Ala Ala Tyr Ala Val Ala Tyr Lys Thr Ala Thr Gly Pro Thr Pro
                   85                  90                  95
   Glu Ala Lys Tyr Asp Ala Phe Val Ala Ala Leu Thr Glu Ala Leu Arg
               100                 105                 110
   Val Ile Ala Gly Ala Leu Glu Val His Ala Val Lys Pro Ala Ala Glu
           115                 120                 125
   Glu Val Pro Ala Ala Lys Ile Pro Ala Gly Glu Leu Gln Ile Val Asp
       130                 135                 140
   Lys Ile Asp Ala Ala Tyr Lys Ile Ala Ala Thr Ala Ala Asn Ala Ala
   145                 150                 155                 160
   Pro Ala Asn Asp Lys Phe Thr Val Phe Glu Gly Ala Phe Asn Lys Ala
                   165                 170                 175
   Ile Lys Glu Ser Thr Gly Gly Ala Tyr Glu Ser Tyr Lys Phe Ile Pro
               180                 185                 190
   Thr Leu Glu Ala Ala Val Lys Gln Ala Tyr Ala Ala Thr Val Ala Ala
           195                 200                 205
   Ala Pro Glu Val Lys Tyr Ala Val Phe Glu Ala Ala Leu Thr Lys Ala
       210                 215                 220
   Ile Thr Ala Met Ser Glu Ala Gln Lys Val Ala Thr Pro Ala Ala Val
   225                 230                 235                 240
   Ala Thr Gly Ala Ala Thr Ala Ala Ala Ser Ala Ala Thr Gly Ala Ala
                   245                 250                 255
   Thr Ala Ala Ala Gly Gly Tyr Lys Val
               260                 265
<210> 5
   <211> 798
   <212> ADN
   <213> Dactylis glomerata
<220>
   <221> CDS
   <222> (1) .. (798)
   <223> Dac g5 séquence mature isoforme 1
<400> 5
   gcc gac gcc ggc tac acc ccg gcc gcc gcg gcc acc ccg gct acc gct    48
   Ala Asp Ala Gly Tyr Thr Pro Ala Ala Ala Ala Thr Pro Ala Thr Ala
    1               5                  10                  15
   gga ggg aag gcg atg acc gag gag cag acg cta atc gag gac gtc aat    96
   Gly Gly Lys Ala Met Thr Glu Glu Gln Thr Leu Ile Glu Asp Val Asn
               20                  25                  30
   gct ggt ttc aag gcg gcc gtg gcc gcc gcc tcc agt gcc cct ccg gcg   144
   Ala Gly Phe Lys Ala Ala Val Ala Ala Ala Ser Ser Ala Pro Pro Ala
           35                  40                  45
   gac aag ttc aag acc ttc gag gcc acc ttc acc gcg gcc tgc aag gct   192
   Asp Lys Phe Lys Thr Phe Glu Ala Thr Phe Thr Ala Ala Cys Lys Ala
       50                  55                  60
   aac atc gcc gcc gcc gcc acc aag gtg ccc ctg ttc gtc gcc aag ctc   240
   Asn Ile Ala Ala Ala Ala Thr Lys Val Pro Leu Phe Val Ala Lys Leu
   65                  70                  75                  80
   gac gcc gcc tac gcc gtc gcc tac aag acc gcc acg ggc ccc acc ccc   288
   Asp Ala Ala Tyr Ala Val Ala Tyr Lys Thr Ala Thr Gly Pro Thr Pro
                   85                  90                  95
   gag gcc aag tac gac gcc ttc gtc gcc gcc ctc acc gaa gcg ctc cgc     336
   Glu Ala Lys Tyr Asp Ala Phe Val Ala Ala Leu Thr Glu Ala Leu Arg
               100                 105                 110
   gtt atc gcc ggc gcc ctc gaa gtc cac gcc gtc aag ccc gct gcc gag     384
   Val Ile Ala Gly Ala Leu Glu Val His Ala Val Lys Pro Ala Ala Glu
           115                 120                 125
   gag gtt ccc gcg gcc aag atc ccc gcc ggt gag ctg cag att gtc gac     432
   Glu Val Pro Ala Ala Lys Ile Pro Ala Gly Glu Leu Gln Ile Val Asp
       130                 135                 140
   aag atc gac gcc gcc tac aag atc gca gcc acc gcc gca aac gcc gcc     480
   Lys Ile Asp Ala Ala Tyr Lys Ile Ala Ala Thr Ala Ala Asn Ala Ala
   145                 150                 155                 160
   ccc gcc aac gac aag ttc acc gtc ttc gag ggc gcc ttc aac aag gcc     528
   Pro Ala Asn Asp Lys Phe Thr Val Phe Glu Gly Ala Phe Asn Lys Ala
                   165                 170                 175
   atc aag gag agc acc ggc ggc gca tac gag agt tac aag ttc atc ccc     576
   Ile Lys Glu Ser Thr Gly Gly Ala Tyr Glu Ser Tyr Lys Phe Ile Pro
               180                 185                 190
   acg ctt gag gcc gcg gtc aag cag gcc tac gcc gcc acc gtg gcc gcc     624
   Thr Leu Glu Ala Ala Val Lys Gln Ala Tyr Ala Ala Thr Val Ala Ala
           195                 200                 205
   gcg ccc gag gtc aag tac gcc gtc ttt gag gcc gcg ctg acc aag gcc     672
   Ala Pro Glu Val Lys Tyr Ala Val Phe Glu Ala Ala Leu Thr Lys Ala
       210                 215                 220
   atc acc gcc atg tcc gag gca cag aag gtc gcc acg cct gcc gcc gtt     720
   Ile Thr Ala Met Ser Glu Ala Gln Lys Val Ala Thr Pro Ala Ala Val
   225                 230                 235                 240
   gct aca ggt gcg gca acc gcc gct gcc agt gct gct acc ggc gct gcc     768
   Ala Thr Gly Ala Ala Thr Ala Ala Ala Ser Ala Ala Thr Gly Ala Ala
                   245                 250                 255
   acc gcc gct gcc ggt ggc tac aaa gtc tga                             798
   Thr Ala Ala Ala Gly Gly Tyr Lys Val
               260                 265
<210> 6
   <211> 265
   <212> PRT
   <213> Dactylis glomerata
<400> 6
   Ala Asp Ala Gly Tyr Thr Pro Ala Ala Ala Ala Thr Pro Ala Thr Ala
   1 5 10 15
   Gly Gly Lys Ala Met Thr Glu Glu Gln Thr Leu Ile Glu Asp Val Asn
   20 25 30
   Ala Gly Phe Lys Ala Ala Val Ala Ala Ala Ser Ser Ala Pro Pro Ala
   35 40 45
   Asp Lys Phe Lys Thr Phe Glu Ala Thr Phe Thr Ala Ala Cys Lys Ala
   50 55 60
   Asn Ile Ala Ala Ala Ala Thr Lys Val Pro Leu Phe Val Ala Lys Leu
   65 70 75 80
   Asp Ala Ala Tyr Ala Val Ala Tyr Lys Thr Ala Thr Gly Pro Thr Pro
   85 90 95
   Glu Ala Lys Tyr Asp Ala Phe Val Ala Ala Leu Thr Glu Ala Leu Arg
   100 105 110
   Val Ile Ala Gly Ala Leu Glu Val His Ala Val Lys Pro Ala Ala Glu
   115 120 125
   Glu Val Pro Ala Ala Lys Ile Pro Ala Gly Glu Leu Gln Ile Val Asp
   130 135 140
   Lys Ile Asp Ala Ala Tyr Lys Ile Ala Ala Thr Ala Ala Asn Ala Ala
   145 150 155 160
   Pro Ala Asn Asp Lys Phe Thr Val Phe Glu Gly Ala Phe Asn Lys Ala
   165 170 175
   Ile Lys Glu Ser Thr Gly Gly Ala Tyr Glu Ser Tyr Lys Phe Ile Pro
   180 185 190
   Thr Leu Glu Ala Ala Val Lys Gln Ala Tyr Ala Ala Thr Val Ala Ala
   195 200 205
   Ala Pro Glu Val Lys Tyr Ala Val Phe Glu Ala Ala Leu Thr Lys Ala
   210 215 220
   Ile Thr Ala Met Ser Glu Ala Gln Lys Val Ala Thr Pro Ala Ala Val
   225 230 235 240
   Ala Thr Gly Ala Ala Thr Ala Ala Ala Ser Ala Ala Thr Gly Ala Ala
   245 250 255
   Thr Ala Ala Ala Gly Gly Tyr Lys Val
   260 265
<210> 7
   <211> 798
   <212> ADN
   <213> Dactylis glomerata
<220>
   <221> CDS
   <222> (1)..(798)
   <223> Dac g5 séquence mature isoforme 2
<400> 7
   gcc gac gcc ggc tac acc ccg gcc gcc gcg gcc acc ccg gct gct gct     48
   Ala Asp Ala Gly Tyr Thr Pro Ala Ala Ala Ala Thr Pro Ala Ala Ala
    1               5                  10                  15
   gga ggg aag gcg atg acc gag gag cag aag cta atc gag gac gtc aac     96
   Gly Gly Lys Ala Met Thr Glu Glu Gln Lys Leu Ile Glu Asp Val Asn
               20                  25                  30
   gct ggc ttc aag gcg gcc gtg gcc gcc gcc tcc agt gcc cct ccg gcg    144
   Ala Gly Phe Lys Ala Ala Val Ala Ala Ala Ser Ser Ala Pro Pro Ala
           35                  40                  45
   gac aag ttc aag acc ttc gag gcc acc ttc acc gcg gcc tgc aag gct    192
   Asp Lys Phe Lys Thr Phe Glu Ala Thr Phe Thr Ala Ala Cys Lys Ala
       50                  55                  60
   aac atc gcc gcc gcc gcc acc aag gtg ccc ctg ttc gtc gcc aag ctc    240
   Asn Ile Ala Ala Ala Ala Thr Lys Val Pro Leu Phe Val Ala Lys Leu
   65                  70                  75                  80
   gac gcc gcc tac gcc gtc gcc tac aag acc gcc gcg ggc ccc acc ccc    288
   Asp Ala Ala Tyr Ala Val Ala Tyr Lys Thr Ala Ala Gly Pro Thr Pro
                   85                  90                  95
   gag gcc aag tac gac gcc ttt gtc gcc gcc ctc acc gaa gca ctc cgc    336
   Glu Ala Lys Tyr Asp Ala Phe Val Ala Ala Leu Thr Glu Ala Leu Arg
               100                 105                 110
   gtt atc gcc ggc gcc ctc gaa gtc cac gcc gtc aag ccc gct gcc gag    384
   Val Ile Ala Gly Ala Leu Glu Val His Ala Val Lys Pro Ala Ala Glu
           115                 120                 125
   gag gtt ccc gcg gcc aag atc ccc gcc ggt gag ctg cag att gtc gac    432
   Glu Val Pro Ala Ala Lys Ile Pro Ala Gly Glu Leu Gln Ile Val Asp
       130                 135                 140
   aag atc gac gcc gcc tac aag atc gca gcc acc gcc gca aac gcc gcc    480
   Lys Ile Asp Ala Ala Tyr Lys Ile Ala Ala Thr Ala Ala Asn Ala Ala
   145                 150                 155                 160
   ccc gcc aac gac aag ttc acc gtc ttc gag ggc gcc ttc aac aag gcc    528
   Pro Ala Asn Asp Lys Phe Thr Val Phe Glu Gly Ala Phe Asn Lys Ala
                   165                 170                 175
   atc aag gag agc acc ggc ggc gca tac gag agt tac aag ttc atc ccc    576
   Ile Lys Glu Ser Thr Gly Gly Ala Tyr Glu Ser Tyr Lys Phe Ile Pro
               180                 185                 190
   acg ctt gag gcc gcg gtc aag cag gcc tac gcc gcc acc gtg gcc gcc    624
   Thr Leu Glu Ala Ala Val Lys Gln Ala Tyr Ala Ala Thr Val Ala Ala
           195                 200                 205
   gcg ccc gag gtc aag tac gcc gtc ttt gag gcc gcg ctg acc aag gcc    672
   Ala Pro Glu Val Lys Tyr Ala Val Phe Glu Ala Ala Leu Thr Lys Ala
       210                 215                 220
   atc acc gcc atg tcc gag gca cag aag gtc gcc acg ccc gcc gcc gct    720
   Ile Thr Ala Met Ser Glu Ala Gln Lys Val Ala Thr Pro Ala Ala Ala
   225                 230                 235                 240
   gct aca ggt gcg gca acc gcc gct gcc agt gct gct acc ggc gct gcc    768
   Ala Thr Gly Ala Ala Thr Ala Ala Ala Ser Ala Ala Thr Gly Ala Ala
                   245                 250                 255
   acc gcc gct gcc ggt ggc tac aaa gtc tga                            798
   Thr Ala Ala Ala Gly Gly Tyr Lys Val
               260                 265
<210> 8
   <211> 265
   <212> PRT
   <213> Dactylis glomerata
<400> 8
   Ala Asp Ala Gly Tyr Thr Pro Ala Ala Ala Ala Thr Pro Ala Ala Ala
    1               5                  10                  15
   Gly Gly Lys Ala Met Thr Glu Glu Gln Lys Leu Ile Glu Asp Val Asn
               20                  25                  30
   Ala Gly Phe Lys Ala Ala Val Ala Ala Ala Ser Ser Ala Pro Pro Ala
           35                  40                  45
   Asp Lys Phe Lys Thr Phe Glu Ala Thr Phe Thr Ala Ala Cys Lys Ala
       50                  55                  60
   Asn Ile Ala Ala Ala Ala Thr Lys Val Pro Leu Phe Val Ala Lys Leu
   65                  70                  75                  80
   Asp Ala Ala Tyr Ala Val Ala Tyr Lys Thr Ala Ala Gly Pro Thr Pro
                   85                  90                  95
   Glu Ala Lys Tyr Asp Ala Phe Val Ala Ala Leu Thr Glu Ala Leu Arg
               100                 105                 110
   Val Ile Ala Gly Ala Leu Glu Val His Ala Val Lys Pro Ala Ala Glu
           115                 120                 125
   Glu Val Pro Ala Ala Lys Ile Pro Ala Gly Glu Leu Gln Ile Val Asp
       130                 135                 140
   Lys Ile Asp Ala Ala Tyr Lys Ile Ala Ala Thr Ala Ala Asn Ala Ala
   145                 150                 155                 160
   Pro Ala Asn Asp Lys Phe Thr Val Phe Glu Gly Ala Phe Asn Lys Ala
                   165                 170                 175
   Ile Lys Glu Ser Thr Gly Gly Ala Tyr Glu Ser Tyr Lys Phe Ile Pro
               180                 185                 190
   Thr Leu Glu Ala Ala Val Lys Gln Ala Tyr Ala Ala Thr Val Ala Ala
           195                 200                 205
   Ala Pro Glu Val Lys Tyr Ala Val Phe Glu Ala Ala Leu Thr Lys Ala
       210                 215                 220
   Ile Thr Ala Met Ser Glu Ala Gln Lys Val Ala Thr Pro Ala Ala Ala
   225                 230                 235                 240
   Ala Thr Gly Ala Ala Thr Ala Ala Ala Ser Ala Ala Thr Gly Ala Ala
                   245                 250                 255
   Thr Ala Ala Ala Gly Gly Tyr Lys Val
               260                 265
<210> 9
   <211> 30
   <212> ADN
   <213> Dactylis glomerata
<220>
   <221> misc_feature
   <222> (1) .. (30)
   <223> amorce sens pro-séquence
<400> 9
   gggtctagaa tggcggtcca gaagtacacc 30
<210> 10
   <211> 30
   <212> ADN
   <213> Dactylis glomerata
<220>
   <221> misc_feature
   <222> (1) .. (30)
   <223> Amorce antisens pro-séquence
<400> 10
   ggggagctct cagactttgt agccaccggc 30
<210> 11
   <211> 33
   <212> ADN
   <213> Dactylis glomerata
<220>
   <221> misc_feature
   <222> (1) .. (33)
   <223> Amorce sens séquence mature
<400> 11
   aagctcgaga aaagagccga cgccggctac acc 33
<210> 12
   <211> 33
   <212> ADN
   <213> Dactylis glomerata
<220>
   <221> misc_feature
   <222> (1) .. (33)
   <223> Amorce antisens séquence mature
<400> 12
   gggggcggcc gctcagactt tgtagccacc ggc 33

## Description

La présente invention a pour objet le clonage et le séquençage d'allergènes de pollen de *Da ctylis glomerata* et plus particulièrement de l'allergène Dac g5. La présente a également pour objet la production de cet allergène recombinant pour être incorporé dans des préparations utiles pour le diagnostic ou le traitement des allergies.

Les allergènes sont les protéines les plus abondantes du pollen et constituent la cause majeure d'allergies dans les climats tempérés. L'allergène objet de la présente invention est Dac g5 du pollen de *Dactylis glomerata.*

Certains individus prédisposés génétiquement deviennent hypersensibles (allergiques) à des antigènes provenant de sources environnementales très variées. Les antigènes capables d'induire une réaction d'hypersensibilisation immédiate ou retardée sont appelés allergènes. Les allergènes peuvent notamment avoir pour origine des arbres, des plantes herbacées, des insectes, des mammifères, de la nourriture, des médicaments ou des produits chimiques. Les allergènes sont classés en groupes de I à V selon leurs propriétés immunochimiques. L'allergène Dac g5 de *Dactylis glomerata* fait partie du groupe V, comme l'allergène Lol pV de *Lolium* perenne.

Les anticorps impliqués dans l'allergie appartiennent à la classe des immunoglobulines de type IgE. En présence d'un allergène, les IgE se lient aux mastocytes et aux basophiles, ce qui conduit au relargage par ces cellules de différents médiateurs chimiques et à la manifestation de l'allergie.

Celle-ci peut prendre différentes formes, comme par exemple un choc anaphylactique, de l'asthme, une rhinite ou une dermatite atopique.

Lorsque le diagnostic de l'allergie à un composé particulier a été établi, la désensibilisation du patient vis-à-vis de l'allergène en cause est l'approche thérapeutique la plus fréquente, notamment lorsque la présence de l'allergène ne peut être évitée comme dans le cas du pollen et des acariens. Ce type de traitement a prouvé son efficacité, mais il nécessite de disposer d'un produit efficace et sûr. En effet, le traitement présente un risque de choc anaphylactique, de plus le produit administré doit être dépourvu de toute impureté qui pourrait constituer un autre allergène potentiel. Or, aujourd'hui, on ne sait utiliser que des mélanges complexes d'allergènes et pas de produits purs. Il est donc nécessaire de pouvoir disposer d'allergènes sous une forme structurale la plus proche possible de l'allergène natif et présentant le plus grand degré de pureté possible.

L'un des moyens possibles pour atteindre ce but est la production d'allergènes recombinants dans un organisme hôte (Laffer, S. et al., J. Allergy Clin. Immunol., Septembre 1996, volume 98, no. 3, pages 652-658).

A titre d'exemples, on peut citer, la demande de brevet publiée sous le No. 819 763 qui décrit la production de l'allergène Der fII modifié. Le brevet européen publié sous le No. 406 286 décrit le clonage d'un allergène majeur du pollen de l'ivraie, Lol p1, et l'expression de ce gène. La demande de brevet publiée sous le No. 473 111 rapporte également la production d'allergènes d'acariens recombinants utilisés pour la désensibilisation. La demande de brevet publiée sous le No. 463 059 concerne des allergènes tirés de l'ambroisie et l'utilisation de ces protéines. Ces documents présentent l'expression des gènes et la production des protéines dans *E. coli.*

Ce système d'expression présente l'inconvénient de ne pas assurer les modifications post-traductionnelles des protéines qui peuvent être réalisées dans les cellules eucaryotes. Par exemple, les protéines produites ne sont pas glycosylées, or la glycosylation de certaines protéines allergènes peut être importante pour leur capacité de fixation aux IgE (Van Ree et al., J. Biol. Chem., 2000, volume 275, pages 11451-11458).

La présente invention concerne le clonage, le séquençage et la préparation d'une molécule d'acide nucléique purifiée comprenant une séquence nucléotidique codant pour la protéine constituant l'allergène Dac g5 ou pour un dérivé de celle-ci. La présente invention concerne encore l'insertion de cet acide nucléique dans un vecteur d'expression et la production de la protéine recombinante dans un organisme ou micro-organisme hôte, en particulier dans des cellules végétales et des plantes ou partie de plantes, et de préférence dans des suspensions cellulaires et des plants de tabac. Cet allergène est produit en vue de son utilisation en diagnostic et en immunothérapie.

La production des allergènes dans des cellules végétales, comme par exemple le tabac, présente l'avantage de permettre la production de protéines recombinantes glycosylées. Ce moyen de production n'a jusqu'à présent jamais été utilisé pour la production d'allergènes.

Les travaux réalisés dans le cadre de la présente invention ont concerné :
- le clonage de l'ADNc de Dac g5,
- l'insertion de la molécule d'acide nucléique clonée dans un vecteur d'expression approprié,
- la production de Dac g5 recombinant dans des systèmes biologiques,
- les tests immunologiques de l'allergène purifié.

Dac g5 est une protéine de 26,5 kDa, de 265 acides aminés, reconnue par au moins 90 % des sujets allergiques au pollen de graminées.

L'allergène Dac g5 de *Dactylis glomerata* appartient aux allergènes du groupe V. Des séquences nucléotidiques codant pour des allergènes homologues à Dac g5 ont été décrites dans l'art antérieur (WO 95/06728). Les inventeurs ont défini des oligonucléotides dégénérées à partir de ces séquences afin d'amplifier spécifiquement par la technique de RT-PCR un ADNc codant Dac g5. Un fragment d'ADNc de taille attendue a été cloné à partir d'une population d'ARN totaux de pollen de *Dactylis glomerata* puis séquencé. Ce fragment a été utilisé pour définir des amorces spécifiques pour les protocoles 5' et 3' RACE-PCR. Ces protocoles ont permis de compléter les extrémités 5' et 3' du fragment. Après amplification des extrémités 5' et 3', l'ADNc complet codant pour Dac g5 a été séquencé.

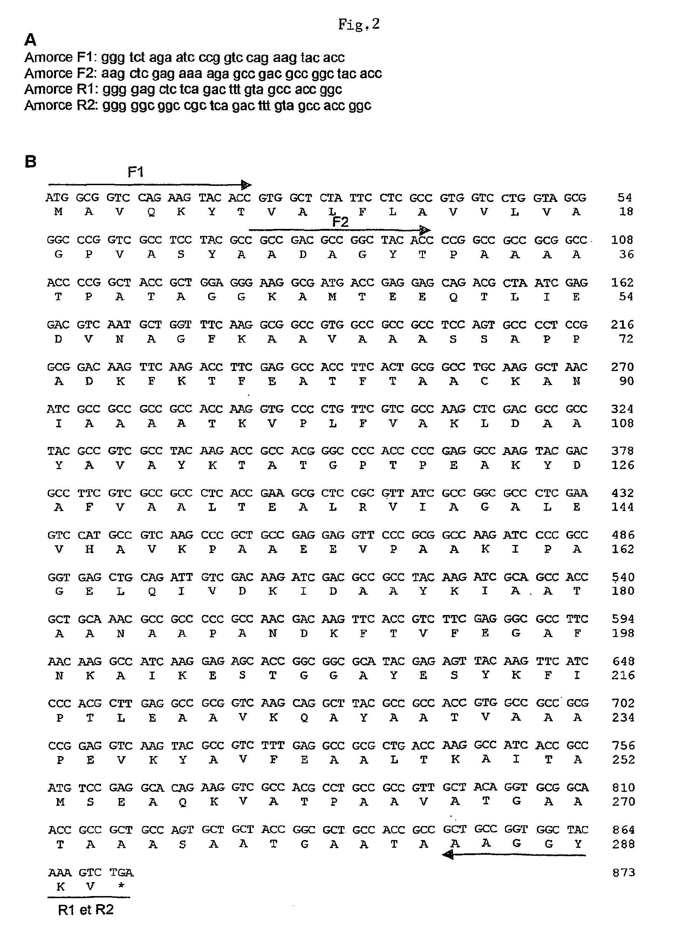

Les séquences obtenues ont permis de définir de nouvelles amorces spécifiques de Dac g5, ces amorces ont été utilisées pour cloner l'ADNc de la pro-forme et de la forme mature de l'allergène. Les séquences nucléotidiques des amorces utilisées pour les réactions de PCR sont les suivantes :
- amorce sens (pro-forme), représentée à la figure 2A (amorce C1) et sous le numéro SEQ ID NO.9 dans la liste de séquences en annexe :
   5'GGG TCT AGA ATG GCG GTC CAG AAG TAC ACC 3'
- amorce antisens, utilisée pour cloner la pro-forme, représentée à la figure 2A (amorce F1) et sous le numéro SEQ ID NO.10 dans la liste de séquences en annexe :
   5'GGG GAG CTC TCA GAC TTT GTA GCC ACC GGC 3'
- amorce sens (forme mature), représentée à la figure 2A (amorce F2) et sous le numéro SEQ ID NO.11 dans la liste de séquences en annexe :
   5'AAG CTC GAG AAA AGA GCC GAC GCC GGC TAC ACC 3'
- amorce antisens, utilisée pour cloner la forme mature, représentée à la figure 2A (amorce R2) et sous le numéro SEQ ID NO.12 dans la liste de séquences en annexe :
   5'GGG GGC GGC CGC TCA GAC TTT GTA GCC ACC GGC 3'

Les clones ADNc ont été séquencés, la séquence en acides aminés correspondant à chaque séquence nucléotidique a été déterminée.

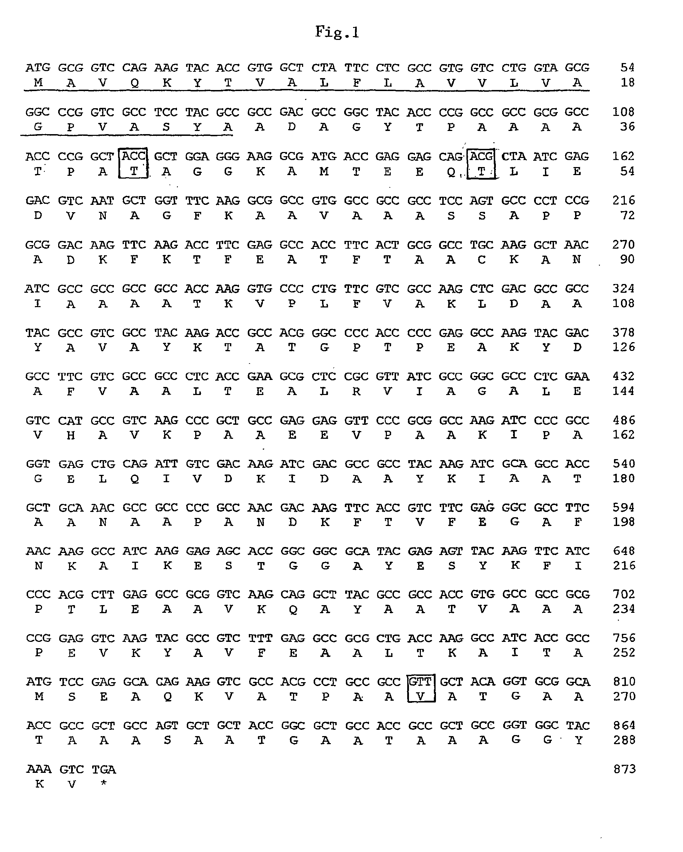

Les inventeurs sont donc maintenant parvenus à cloner et séquencer l'acide nucléique codant pour l'allergène Dac g5 du pollen de *Dactylis glomerata.* En conséquence, l'invention concerne une molécule d'acide nucléique purifiée constituée par ou comprenant une séquence nucléotidique codant pour l'allergène Dac g5 ou un dérivé de celle-ci. La séquence en acide aminé de la pro-forme de l'allergène Dac g5 est représentée à la figure 1 et sous le numéro SEQ ID NO.2 dans la liste de séquences en annexe. Un fragment de cette séquence, délimité par les acides aminés en position 25 à 290, constitue la protéine mature Dac g5. La séquence en acide aminé de la forme mature de l'allergène Dac g5 est représentée à la figure 1 et sous le numéro SEQ ID NO.4 dans la liste de séquences en annexe.

La figure 1 en annexe donne la séquence nucléotidique et peptidique de la pro-forme de l'allergène Dac g5 (isoforme 1). La séquence soulignée correspond à la séquence signal absente de la protéine mature. Les codons et les acides aminés qui diffèrent dans l'isoforme 2 sont encadrés. Le tableau 1 ci-après indique les variations observées entre les isoformes 1 et 2.

**Tableau 1**

| Position des acides aminés | Isoforme 1 | Isoforme 2 |
|---|---|---|
| 40 | Thr (ACC) | Ala (GCT) |
| 51 | Thr (ACG) | Lys (AAG) |
| 265 | Val (GTT) | Ala (GCT) |

La figure 2 représente en A la séquence des amorces, et en B la séquence de la pro-forme de l'allergène Dac g5 et le positionnement des amorces sur cette séquence.

On entend par dérivé de la protéine constituant l'allergène Dac g5, une protéine dont la séquence en acides aminés diffère par la modification, la suppression ou l'addition d'un ou plusieurs acides aminés dès lors que cette protéine est fonctionnellement et/ou immunologiquement équivalente à Dac g5.

De telles modifications peuvent résulter de la dégénérescence du code génétique ou de modifications de la séquence en acide nucléique par toute technique de biologie moléculaire. L'homme du métier est à même de déterminer parmi ces séquences celles qui présentent des propriétés fonctionnelles et immunologiques identiques ou proches à Dac g5 par exemple à l'aide d'anticorps. A ce titre, les inventeurs ont cloné deux isoformes de Dac g5. Les séquences en acide aminé de ces isoformes matures de l'allergène Dac g5 sont représentées sous le numéro SEQ ID NO.6 et SEQ ID NO.8 dans la liste de séquence en annexe. L'invention envisage donc également les isoformes de la protéine constituant l'allergène Dac g5 et présentant une homologie de séquences en acides aminés supérieure à 50% de préférence supérieure à 70% et tout préférentiellement supérieure à 90% avec la séquence représentée sous le numéro SEQ ID NO.2 en annexe. L'invention envisage plus particulièrement une protéine, dérivé fonctionnel et immunologiquement équivalent à Dac g5, dont la séquence en acides aminés est choisie parmi les séquences SEQ ID NO.6 et SEQ ID NO.8 dans la liste de séquence en annexe.

On entend par fragment de la protéine constituant l'allergène Dac g5, tout peptide ou polypeptide issu de la protéine, plus particulièrement utile pour le diagnostic de l'allergie.

Une molécule d'acide nucléique purifiée comprenant ou constituée par une séquence nucléotidique (ADNc) codant pour la pro-forme de l'allergène Dac g5 est représentée sous le numéro SEQ ID NO.1 dans la liste de séquences en annexe. L'invention concerne aussi un dérivé, et plus particulièrement une molécule d'acide nucléique codant pour la protéine mature qui est délimitée par les nucléotides en position 75 à 870 de la séquence nucléotidique représentée sous le numéro SEQ ID NO.1 dans la liste de séquences en annexe. Cette séquence est représentée sous le numéro SEQ ID NO.3 dans la liste de séquences en annexe.

L'invention concerne également les molécules d'acide nucléique codant des isoformes de la protéine Dac g5 mature et plus particulièrement celles dont les séquences en acides aminés sont représentées sous les numéros SEQ ID NO.6 et SEQ ID NO.8 dans la liste de séquences en annexe.

A partir de la séquence nucléotidique codant pour Dac g5 ou une de ses isoformes, l'homme du métier peut définir des séquences nucléotidiques codant pour des protéines ou polypeptides correspondant à un fragment de Dac g5 ou d'une de ses isoformes et possédant par exemple au moins un épitope de Dac g5 ou un épitope d'une des isoformes de Dac g5. Il peut également définir une séquence nucléotidique codant pour des protéines équivalentes fonctionnellement à Dac g5 ou à une de ses isoformes mais dont la séquence en acides aminés n'est pas identique à celle de Dac g5 ou à une de ses isoformes.

Il peut enfin définir une séquence nucléotidique codant pour des protéines équivalentes à Dac g5 ou à une de ses isoformes sur le plan immunologique. Ces protéines sont, par exemple, capables de se lier à des anticorps anti-Dac g5, mais ne possèdent pas la fonction enzymatique de l'allergène Dac g5 natif. On entend plus particulièrement par dérivé d'une molécule d'acide nucléique selon l'invention, une molécule d'acide nucléique capable de s'hybrider dans des conditions d'hybridation standards avec l'une des séquences nucléotidiques représentées sous les numéros SEQ ID NO.1 et SEQ ID NO.3 dans la liste de séquences en annexe. Il s'agit par exemple des séquences nucléotidiques codant les isoformes de Dac g5 représentées sous les numéros SEQ ID NO.5 et SEQ ID NO.7 dans la liste de séquences en annexe.

Il est décrit également la mutagenèse de la protéine permettant d'introduire en certaines positions définies de la protéine un ou plusieurs sites portant une fonction particulière. Il peut s'agir par exemple d'introduire un site de N-glycosylation sur l'allergène.

L'invention se rapporte aussi à une molécule d'acide nucléique recombinante comprenant une séquence polynucléotidique codant pour l'allergène Dac g5 ou une de ses isoformes ou un dérivé de ceux-ci fonctionnel et/ou immunologique de la protéine Dac g5 ou d'une de ses isoformes, un promoteur lié de manière fonctionnelle à ladite séquence, éventuellement un gène de sélection placé sous le contrôle de son propre promoteur ou du même promoteur que ladite séquence, et avantageusement une séquence de terminaison placée en aval de ladite séquence. Il peut s'agir d'une cassette ou de préférence d'un vecteur.d'expression comprenant notamment une origine de réplication eucaryote ou procaryote, une séquence promotrice adaptée, un marqueur de sélection et une séquence nucléotidique codant pour l'allergène Dac g5 ou une de ses isoformes, ou un dérivé de ceux-ci placé sous le contrôle desdites séquences de régulation. L'homme du métier choisira sans difficulté parmi les vecteurs d'expression connus dans l'art antérieur le vecteur le mieux adapté à l'organisme hôte dans lequel il envisage de faire produire la protéine.

L'invention envisage tout particulièrement pour la production de la protéine constituant l'allergène Dac g5 ou une de ses isoformes, un vecteur permettant l'expression de l'acide nucléique dans des cellules eucaryotes, et de préférence dans des cellules végétales ou des levures.

Le vecteur d'expression peut également être construit de manière à permettre la production de la protéine recombinante définie précédemment sous la forme d'une protéine de fusion. Le polypeptide fusionné à la protéine d'intérêt peut notamment être utile pour permettre ou faciliter la purification de celle-ci. Ce polypeptide peut en particulier être une séquence constituée de plusieurs histidines ou « Histidine-Tag » ajoutée dans une région variable non critique pour l'activité et la conformation de la molécule, il peut s'agir d'une région interne ou d'une extrémité N- ou C-terminale. L'adjonction d'une séquence "Histidine-Tag" permet de purifier la protéine recombinante par chromatographie d'affinité sur une colonne de métal chélaté.

L'invention se rapporte aussi à un hôte, eucaryote ou procaryote, transformé par un vecteur d'expression comme défini ci-dessus. Cet hôte peut par exemple être *E*. *coli, Saccharomyces cerevisiae, Pichia Pastoris,* ou une cellule végétale notamment une cellule de *Nicotiana tabacum* dans le génome de laquelle est incorporée de façon stable la molécule d'acide nucléique codant pour l'allergène Dac g5 ou une de ses isoformes ou un dérivé de ceux-ci.

La présente invention concerne encore un organisme ou micro-organisme, de préférence une cellule ou une plante, tout préférentiellement de tabac, ayant incorporé dans son génome, avantageusement de manière stable, une molécule d'acide nucléique de l'invention placé sous le contrôle de séquence de régulation de manière à exprimer l'allergène Dac g5 ou une de ses isoformes dans ces cellules, dans une plante ou une partie déterminée de la plante.

Des plantes transgéniques selon l'invention peuvent être préparées en transformant une cellule végétale avec ladite molécule d'acide nucléique puis en régénérant une plante à partir de la cellule transformée.

L'invention concerne un procédé d'obtention de la protéine Dac g5 recombinante ou d'une de ses isoformes ou d'une de ses isoformes, ou d'un dérivé fonctionnel ou immunologique de Dac g5 ou d'une de ses isoformes. Ce procédé comprend la culture d'un organisme, procaryote ou eucaryote, transformé par un vecteur d'expression tel que défini précédemment, dans des conditions et pendant un temps suffisant pour permettre l'expression de ladite protéine.

Ce procédé comprend également l'isolement des protéines produites à partir de la culture des organismes transformés. Dans le cas particulier de l'expression de protéines recombinantes dans des cellules de tabac, les cals exprimant l'allergène d'intérêt sont sélectionnés par immunodétection à l'aide d'un anticorps dirigé contre la forme naturelle de Dac g5. Les allergènes sont localisés par fractionnement cellulaire, puis ils sont purifiés à partir de suspensions cellulaires transgéniques par immunodétection à l'aide d'un anticorps dirigé contre la forme naturelle de Dac g5. Le procédé selon l'invention comprend également l'analyse structurale et immunologique de la ou des protéines produites.

Il est décrit donc aussi l'allergène Dac g5 ou un dérivé de celui-ci recombinant obtenu par ledit procédé. L'invention concerne la pro-forme et la forme mature de l'allergène Dac g5. L'invention concerne les isoformes de la forme mature de Dac g5. aussi des fragments peptidiques ou polypeptidiques de Dac g5 ou d'une de ses isoformes, ainsi que des protéines fonctionnellement ou immunologiquement équivalentes à Dac g5 ou à une de ses isoformes. Les protéines équivalentes à Dac g5 ou à une de ses isoformes peuvent être obtenues notamment par mutagenèse dirigée appliquée à la molécule d'ADN codant pour Dac g5 ou pour une de ses isoformes. également une protéine de fusion recombinante comprenant la protéine Dac g5 ou une de ses isoformes, un fragment de ces protéines ou un équivalent fonctionnel ou immunologique.

Cet allergène recombinant, un dérivé de celui-ci, comme l'allergène naturel, peuvent être utilisés pour préparer des anticorps monoclonaux ou polyclonaux. Les anticorps monoclonaux sont préparés selon des techniques classiques, bien connues de l'homme du métier. Des anticorps polyclonaux peuvent être obtenus en immunisant des animaux avec l'allergène Dac g5 à l'aide d'un adjuvant adapté, les anticorps sont ensuite purifiés du sérum des animaux immunisés.

Ces anticorps peuvent notamment servir à détecter la présence de l'allergène Dac g5, d'un fragment peptidique de la protéine ou d'une de ses isoformes ou d'un équivalent immunologique de celles-ci, dans un milieu particulier comme par exemple un milieu de culture.

L'invention concerne en outre les compositions pharmaceutiques destinées au traitement et/ou au diagnostic d'une allergie et comprenant comme principe actif une quantité efficace de Dac g5, il est décrit d'une des isoformes de la protéine ou d'un dérivé fonctionnel ou immunologique de celles-ci, ou encore un anticorps dirigé contre celles-ci. Dans ces compositions, le principe actif est associé à un véhicule pharmaceutiquement acceptable. Les compositions destinées au traitement de l'allergie sont formulées selon des principes adaptés et connus de l'homme du métier afin d'être injectées par voie sous cutanée ou par toute autre voie d'administration.

L'invention concerne également un procédé de détection de la sensibilité manifestée par un individu au pollen d'herbacées et en particulier au pollen de *Dactylis glomerata.* Ce procédé comprend la détection de la présence d'anticorps liant une des isoformes de la protéine recombinante Dac g5 ou un dérivé fonctionnel et/ou immunologique de celles-ci, ou un anticorps spécifique de celles-ci. Ce procédé comprend notamment une étape pendant laquelle un échantillon provenant de l'individu est mis en contact avec une des isoformes de la protéine recombinante Dac g5, un dérivé de celles-ci ou un anticorps dirigée contre ceux-ci, dans des conditions permettant la formation d'un complexe antigène / anticorps, puis la détection dudit complexe.

L'invention concerne enfin un réactif de diagnostic d'une allergie caractérisé en ce qu'il comprend une préparation contenant une des isoformes de la protéine recombinante Dac g5 et/ou un dérivé d'une des isoformes de Dac g5, ou un anticorps dirigé contre celles-ci.

## Revendications

1. Une molécule d'acide nucléique purifiée **caractérisée en ce qu'**elle comprend ou est constituée par une séquence nucléotidique codant pour :
i) l'allergène Dac g5 dont la séquence en acides aminés est représentée sous le numéro SEQ ID NO. 2 dans la liste de séquences en annexe ; ou
ii) une des isoformes matures de l'allergène Dac g5 dont la séquence en acides aminés est choisie parmi les séquences SEQ ID NO. 4, SEQ ID NO.6 ou SEQ ID NO.8 dans la liste de séquences en annexe, ou un dérivé fonctionnel et/ou immunologique de celui-ci présentant une homologie de séquences en acides aminés supérieure à 90% avec la séquence présentée sous le numéro SEQ ID NO :2 en annexe et capable de se lier à des anticorps anti-Dac g5.

2. Une molécule d'acide nucléique selon la revendication 1, **caractérisée en ce qu'**elle comprend ou est constituée par une séquence nucléotidique choisie parmi les séquences SEQ ID NO.1, SEQ ID NO.3, SEQ ID NO.5 ou SEQ ID NO.7 dans la liste de séquences en annexe.

3. Une molécule d'acide nucléique recombinant, **caractérisée en ce qu'**elle comprend une molécule d'acide nucléique selon l'une quelconque des revendications 1 ou 2, un promoteur lié de manière fonctionnelle à ladite molécule d'acide nucléique, éventuellement un gène de sélection placé sous le contrôle de son propre promoteur ou du même promoteur que ladite molécule d'acide nucléique, et avantageusement une séquence de terminaison placée en aval de ladite molécule d'acide nucléique.

4. Un vecteur d'expression **caractérisé en ce qu'**il comprend une origine de réplication eucaryote ou procaryote, une séquence promotrice adaptée, un marqueur de sélection et une molécule d'acide nucléique selon l'une quelconque des revendications 1 à ou 2, placée sous le contrôle desdites séquences de régulation.

5. Un hôte eucaryote non-humain ou procaryote transformé par une molécule d'acide nucléique selon la revendication 3, ou par un vecteur d'expression selon la revendication 4.

6. Un hôte selon la revendication 5, **caractérisé en ce qu'**il est constitué par une cellule végétale dans le génome de laquelle est incorporée, avantageusement de façon stable, une molécule d'acide nucléique selon l'une quelconque des revendications 1 à 3.

7. Une plante et tout préférentiellement une plante de tabac, ayant incorporé de manière stable dans son génome une molécule d'acide nucléique, selon l'une quelconque des revendications 1 ou 2, placée sous le contrôle de séquence de régulation de manière à exprimer l'allergène Dac g5 dans une partie déterminée de la plante.

8. Une suspension cellulaire, tout préférentiellement de tabac, dont les cellules ont incorporé de manière stable dans leur génome une molécule d'acide nucléique, selon l'une quelconque des revendications 1 ou 2, placée sous le contrôle de séquence de régulation de manière à exprimer l'allergène Dac g5 dans lesdites cellules.

9. Une levure ayant incorporé de manière stable dans son génome une molécule d'acide nucléique selon l'une quelconque des revendications 1 ou 2 placée sous le contrôle de séquence de régulation de manière à exprimer l'allergène Dac g5.

10. Un procédé d'obtention de la protéine Dac g5 recombinante, d'une isoforme ou d'un dérivé fonctionnel ou immunologique de celle-ci, tels que définis dans la revendication 1, **caractérisé en ce qu'**il comprend la culture d'un organisme, procaryote ou eucaryote, transformé par une molécule d'acide nucléique selon la revendication 3, ou par un vecteur d'expression selon la revendication 4, dans des conditions et pendant un temps suffisant pour permettre l'expression de ladite protéine, puis l'isolement des protéines produites à partir de la culture des organismes transformés.

11. Une protéine recombinante comprenant ou constituée par l'allergène Dac g5 de séquence SEQ ID NO : 2, une isoforme de celui-ci de séquence choisie parmi les séquences SEQ ID NO : 4, SEQ ID NO : 6 ou SEQ ID NO : 8, ou un dérivé de celui-ci présentant une homologie de séquences en acides aminés supérieure à 90% avec la séquence présentée sous le numéro SEQ ID NO : 2 et capable de se lier à des anticorps anti-Dac g5.

12. Une composition pharmaceutique pour le traitement ou le diagnostic d'une allergie, **caractérisée en ce qu'**elle comprend comme principe une quantité efficace d'une protéine selon la revendication 11 ou d'un anticorps obtenu par immunisation d'un animal avec cette protéine et dirigé contre cette protéine.

13. Un procédé *in vitro* de détection de la sensibilité manifestée par un individu au pollen d'herbacées et en particulier au pollen de *Dactylis glomerata,* **caractérisé en ce que** qu'il comprend la mise en contact d'un échantillon provenant d'un individu avec la protéine selon la revendication 11 ou un anticorps obtenu par immunisation d'un animal avec cette protéine et dirigé contre cette protéine, dans des conditions permettant la formation d'un complexe antigène/anticorps, puis la détection dudit complexe.

14. Un réactif de diagnostic d'une allergie, **caractérisé en ce qu'**il comprend une protéine selon la revendication 11 ou un anticorps obtenu par immunisation d'un animal avec cette protéine et dirigé contre cette protéine.

## Claims

1. A purified nucleic acid molecule, **characterised in that** it comprises or consists of a nucleotide sequence coding for:
i) the Dac g5 allergen, the amino acid sequence of which is represented under the number SEQ ID NO: 2 in the appended list of sequences; or
ii) one of the mature isoforms of the Dac g5 allergen the amino acid sequence of which is chosen from the sequences SEQ ID NO: 4, SEQ ID NO: 6 or SEQ ID NO: 8 in the appended list of sequences, or a functional and/or immunological derivative thereof having a homology of amino acid sequences greater than 90% with the appended sequence presented under the number SEQ ID NO: 2 and capable of bonding to anti-Dac g5 antibodies.

2. A nucleic acid molecule according to claim 1, **characterised in that** it comprises or consists of a nucleotide sequence chosen from the sequences SEQ ID NO: 1, SEQ ID NO: 3, SEQ ID NO: 5 or SEQ ID NO: 7 in the appended list of sequences.

3. A recombinant nucleic acid molecule, **characterised in that** it comprises a nucleic acid molecule according to either one of claims 1 or 2, a promoter functionally linked to said nucleic acid molecule, possibly a selection gene placed under the control of its own promoter or the same promoter as said nucleic acid molecule, and advantageously a termination sequence placed downstream of said nucleic acid molecule.

4. An expression vector, **characterised in that** it comprises a eukaryotic or prokaryotic replication origin, an adapted promoter sequence, a selection marker and a nucleic acid molecule according to either one of claims 1 or 2, placed under the control of said regulation sequences.

5. A prokaryotic or non-human eukaryotic host transformed by a nucleic acid molecule according to claim 3, or by an expression vector according to claim 4.

6. A host according to claim 5, **characterised in that** it consists of a vegetable cell in the genome of which a nucleic acid molecule according to any one of claims 1 to 3 is incorporated, advantageously in a stable manner.

7. A plant or quite preferentially a tobacco plant that has, in a stable manner, incorporated in its genome a nucleic acid molecule, according to either one of claims 1 or 2, placed under the control of a regulation sequence so as to express the Dac g5 allergen in a given part of the plant.

8. A cell suspension, quite preferentially of tobacco, the cells of which have in a stable manner incorporated in their genome a nucleic acid molecule according to either one of claims 1 or 2, placed under the control of a regulation sequence so as to express the Dac g5 allergen in said cells.

9. A yeast that in a stable manner has incorporated in its genome a nucleic acid molecule according to either one of claims 1 or 2 placed under the control of a regulation sequence so as to express the Dac g5 allergen.

10. A method of obtaining the recombinant Dac g5 protein, an isoform or a functional or immunological derivative thereof, as defined in claim 1, **characterised in that** it comprises the culture of an organism, prokaryotic or eukaryotic, transformed by a nucleic acid molecule according to claim 3, or by an expression vector according to claim 4, under conditions and for a sufficient time to allow the expression of said protein, and the the isolation of protein produced from the culture of the transformer organisms.

11. A recombinant protein comprising or consisting of the Dac g5 allergen of sequence SEQ ID NO: 2, an isoform thereof of sequence chosen from the sequences SEQ ID NO: 4, SEQ ID NO: 6 or SEQ ID NO: 8, or a derivative thereof having a homology of amino acid sequences greater than 90% with the sequence presented under the number SEQ ID NO: 2 and capable of bonding to anti-Dac g5 antibodies.

12. A pharmaceutical compositions for the treatment or diagnosis of an allergy, **characterised in that** it comprises as a principle an effective quantity of a protein according to claim 11 or an antibody obtained by immunisation of an animal with this protein and directed against this protein.

13. An *in vitro* method for detecting the sensitivity manifested by an individual to herbaceouus pollen and in particular to the *Dactylis glomerata* pollen, **characterised in that** it comprises putting a sample coming from an individual in contact with the protein according to claim 11 or an antibody obtained by immunization of an animal with this protein and directed against this protein, under conditions allowing the formation of an antigen/antibody complexe, and then the detection of said complex.

14. An allergy diagnosis reagent, **characterised in that** it comprises a protein according to claim 11 or an antibody obtained by immunisation of an animal with this protein and directed against this protein.

## Patentansprüche

1. Gereinigtes Nukleinsäuremolekül, **dadurch gekennzeichnet, dass** es eine Nukieotidsequenz, die für
i) das Allergen Dac g5, dessen Aminosäuresequenz unter der Nummer SEQ ID NO: 2 in der anhängenden Sequenzenliste dargestellt ist, oder
ii) eine der reifen Isoforme des Allergens Dac g5, deren Aminosäuresequenz aus den Sequenzen SEQ ID NO: 4, SEQ ID NO: 6 oder SEQ ID NO: 8 der anhängenden Sequenzenliste ausgewählt ist, oder ein funktionelles und/oder immunologisches Derivat davon, welches eine Sequenzhomologie der Aminosäuren von über 90 % mit der unter der Nummer SEQ ID NO: 2 im Anhang dargestellten Sequenz aufweist und in der Lage ist, an Anti-Dac g5-Antikörper zu binden,
kodiert, umfasst oder aus dieser besteht.

2. Nukleinsäuremolekül gemäß Anspruch 1, **dadurch gekennzeichnet, dass** es eine Nukleotidsequenz, die aus den Sequenzen SEQ ID NO: 1, SEQ ID NO: 3, SEQ ID NO:5 oder SEQ ID NO: 7 der anhängenden Sequenzenliste ausgewählt ist, umfasst oder aus dieser besteht.

3. Rekombinantes Nukleinsäuremolekül, **dadurch gekennzeichnet, dass** es ein Nuklesnsäuremolekül gemäß irgendeinem der Ansprüche 1 oder 2, einen Promotor, der funktionell mit dem Nukleinsäuremolekül verbunden ist, gegebenenfalls ein Selektionsgen, welches unter der Kontrolle seines eigenen Promotors oder desselben Promotors wie das Nukleinsäuremolekül platziert ist, und, vorteilhafterweise, eine Terminationssequenz, die stromabwärts des Nukleinsäuremoleküls platziert ist, umfasst.

4. Expressionsvektor, **dadurch gekennzeichnet, dass** er einen eukaryotischen oder prokaryotischen Replikationsursprung (Origin), eine passende Promotorsequenz, einen Selektionsmarker und ein Nukleinsäuremolekül gemäß irgendeinem der Ansprüche 1 oder 2, welches unter der Kontrolle dieser Regulationssequenzen platziert ist, umfasst.

5. Nicht-humaner eukaryotischer oder prokaryotischer Wirt, der mit einem Nukleinsäuremolekül gemäß Anspruch 3 oder mit einem Expressionsvektor gemäß Anspruch 4 transformiert ist.

6. Wirt gemäß Anspruch 5, **dadurch gekennzeichnet, dass** er aus einer Pflanzenzeile besteht, in deren Genom ein Nukleinsäuremolekül gemäß irgendeinem der Ansprüche 1 bis 3, vorteilhafterweise stabil, integriert ist.

7. Pflanze, bevorzugt eine Tabakpflanze, in deren Genom ein Nukleinsäuremolekül gemäß irgendeinem der Ansprüche 1 oder 2, welches unter der Kontrolle von Regulationssequenzen derart platziert ist, dass das Allergen Dac g5 in einem bestimmten Teil der Pflanze exprimiert wird, stabil integriert ist.

8. Zellsuspension, bevorzugt von Tabak, in deren Zellen ein Nukleinsäuremolekül gemäß irgendeinem der Ansprüche 1 oder 2, welches unter der Kontrolle von Regulationssequenzen derart platziert ist, dass das Allergen Dac g5 in den Zellen exprimiert wird, stabil in das Genom integriert ist.

9. Hefe, in deren Genom ein Nukleinsäuremolekül gemäß irgendeinem der Ansprüche 1 oder 2, welches unter der Kontrolle von Regulationssequenzen derart platziert ist, dass das Allergen Dac g5 exprimiert wird, stabil integriert ist.

10. Verfahren zum Erhalt des rekombinanten Dac g5-Proteins, einer Isoform oder eines funktionellen oder immunologischen Derivats davon, wie in Anspruch 1 definiert, **dadurch gekennzeichnet, dass** es das Kultivierten eines prokaryotischen oder eukaryotischen Organismus, das Transformierten mit einem Nukleinsäuremolekül gemäß Anspruch 3 oder mit einem Expressionsvektor gemäß Anspruch 4 unter Bedingungen und für einen Zeitraum, die ausreichend sind, um die Expression des Proteins zu ermöglichten, und anschließendes Isolieren der hergestellten Proteine aus der Kultur der transformierten Organismen umfasst.

11. Rekombinanter Protein, welches das Allergen Dac g5 der Sequenz SEQ ID NO: 2, eine Isoform davon, welche eine aus den Sequenzen SEQ ID NO: 4, SEQ ID NO: 6 oder SEQ ID NO: 8 ausgewählte Sequenz aufweist, oder ein Derivat davon, welches eine Sequenzhomologie der Aminosäuren von über 90 % mit der unter der Nummer SEQ ID NO: 2 dargestellten Sequenz aufweist und in der Lage ist, an Anti-Dac g5-Antikörper zu binden, umfasst oder aus diesen besteht.

12. Pharmazeutische Zusammensetzung zur Behandlung oder Diagnose einer Allergie, **dadurch gekennzeichnet, dass** sie als Wirkstoff eine wirksame Menge eines Proteins gemäß Anspruch 11 oder einen Antikörper, der durch Immunisierung eines Tieres mit diesem Protein verhalten wurde und gegen dieses Protein gerichtet ist, enthält.

13. *In*-*vitro*-Verfahren zum Nachweis der manifestierten Sensibilität eines Individuums gegenüber Gräserpollen, insbesondere gegenüber Pollen von *Dactylis glomerata,* **dadurch gekennzeichnet, dass** es das In-Kontakt-Bringen einer von einem Individuum stammenden Probe mit dem Protein gemäß Anspruch 11 oder einem Antikörper, der durch Immunisierung eines Tieres mit diesem Protein erhalten wurde und gegen dieses Protein gerichtet ist, unter Bedingungen, welche die Bildung eines Antigen-Antikörper-Komplexes ermöglichten, und den anschließenden Nachweis dieses Komplexes umfasst.

14. Reagenz zur Diagnose einer Allergie, **dadurch gekennzeichnet, dass** es ein Protein gemäß Anspruch 11 oder einen Antikörper, der durch Immunisierung eines Tieres mit diesem Protein erhalten wurde und gegen dieses Protein gerichtet ist, umfasst.
